# EUROPEAN PATENT APPLICATION

(11) **EP 4 623 795 A1**
(43) Date of publication of application: **01.10.2025**
(21) Application number: 25166301.9
(22) Date of filing: 26.03.2025
(51) Int. Cl.: A61B 1/00, A61B 1/005

(54) **OPERATING DEVICE FOR ENDOSCOPE AUXILIARY TOOL**

(30) Priority: 29.03.2024 JP 2024057299
(71) Applicant: FUJIFILM Corporation, Tokyo 106-8620 (JP)
(72) Inventor: ONO, Hirotoshi, Kaisei-machi, Ashigarakami-gun, Kanagawa, 2588538 (JP)
(74) Representative: HGF

(57) **Abstract**

An operating device for an endoscope auxiliary tool includes: a first operating member having one end side connected to the endoscope auxiliary tool that is mounted on an insertion part of an endoscope; a rotational movement member that is rotationally movable and that is connected to the other end side of the first operating member; an accommodating member configured to accommodate the rotational movement member; and a second operating member that is provided to protrude from the accommodating member and that is connected to the rotational movement member. A protruding end of the second operating member is configured to be movable along a portion of a circumference of a virtual circle centered on a rotational movement axis of the rotational movement member, and the accommodating member has a grippable region inside the virtual circle.

## Description

### BACKGROUND OF THE INVENTION

### 1. Field of the Invention

The technology of the present disclosure relates to an operating device for an endoscope auxiliary tool.

### 2. Description of the Related Art

JP2019-76672A, JP2014-87489A, JP1993-3851A (JP-H5-3851A), JP2013-106713A, JP1985-99223A (JP-S60-99223A), and JP2015-2858A disclose a technology related to an endoscope.

### SUMMARY OF THE INVENTION

In the technology of the present disclosure, an operating device for an endoscope auxiliary tool having high operability is provided.

An operating device for an endoscope auxiliary tool according to one aspect of the present disclosure comprises: a first operating member having one end side connected to an endoscope auxiliary tool that is mounted on an insertion part of an endoscope; a rotational movement member that is rotationally movable and that is connected to the other end side of the first operating member; an accommodating member that accommodates the rotational movement member; and a second operating member that is provided to protrude from the accommodating member and that is connected to the rotational movement member, in which a protruding end of the second operating member is configured to be movable along a portion of a circumference of a virtual circle centered on a rotational movement axis of the rotational movement member, and the accommodating member has a grippable region inside the virtual circle.

According to the technology of the present disclosure, it is possible to provide an operating device for an endoscope auxiliary tool having high operability.

### BRIEF DESCRIPTION OF THE DRAWINGS

Fig. 1 is a diagram showing a schematic configuration of an endoscope apparatus 100 which is one aspect of a technology of the present disclosure.
Fig. 2 is a perspective view showing an auxiliary device 20 for an endoscope 1 shown in Fig. 1.
Fig. 3 is an enlarged view of the vicinity of a bendable body 30 and a support member 50 in the auxiliary device 20 shown in Fig. 2.
Fig. 4 is a perspective view showing a state where the bendable body 30 and the support member 50 are separated from each other.
Fig. 5 is an exploded perspective view of the bendable body 30.
Fig. 6 is a diagram showing a state where the bendable body 30 is supported by the support member 50 at an end part 10As.
Fig. 7 is a side view of the endoscope 1 and the auxiliary device 20 in Fig. 6 as viewed in a direction of an arrow A.
Fig. 8 is a perspective view of an operating device 40 as viewed from a front side.
Fig. 9 is a perspective view of the operating device 40 as viewed from a rear side.
Fig. 10 is a plan view of the operating device 40.
Fig. 11 is a front view of the operating device 40.
Fig. 12 is a schematic diagram (part 1) showing an example of an operation method of the operating device 40.
Fig. 13 is a schematic diagram (part 2) showing an example of an operation method of the operating device 40.
Fig. 14 is a diagram showing a state where a rear cover member 42B in Fig. 9 is removed.
Fig. 15 is a perspective view showing an inside of the rear cover member 42B.
Fig. 16 is a front view of the operating device 40 in a state shown in Fig. 12.
Fig. 17 is a front view of the operating device 40 in a state shown in Fig. 13.

### DESCRIPTION OF THE PREFERRED EMBODIMENTS

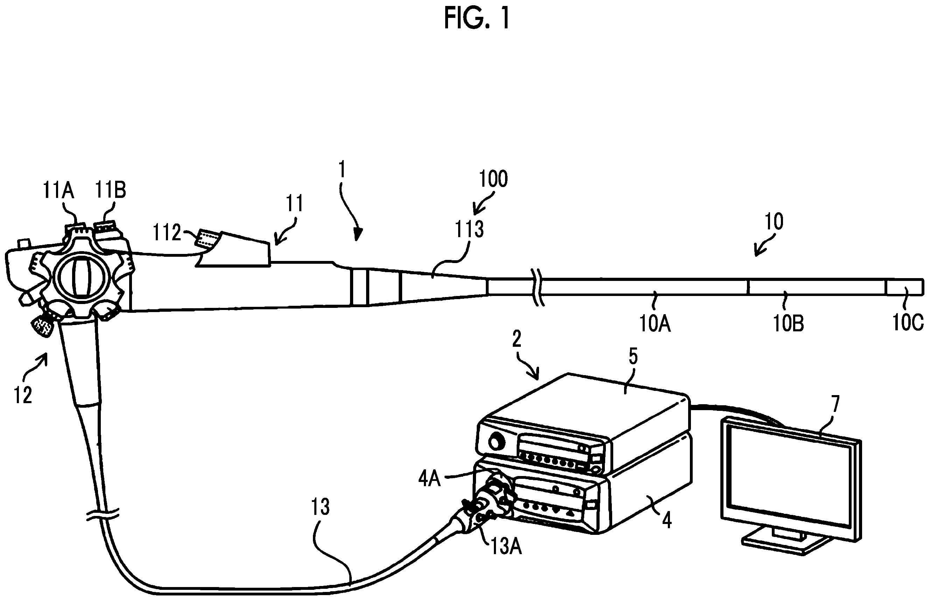

Fig. 1 is a diagram showing a schematic configuration of an endoscope apparatus 100 which is one aspect of a technology of the present disclosure. The endoscope apparatus 100 comprises an endoscope 1, a body part 2 consisting of a light source device 4 and a processor device 5 to which the endoscope 1 is connected, and a display device 7 that displays a captured image obtained by imaging with the endoscope 1, and the like.

The endoscope 1 comprises an insertion part 10 that is an elongated instrument extending in one direction and that is to be inserted into a subject, an endoscope operating part 11 that is provided on a base end side of the insertion part 10 and that is provided with an operating member for performing an observation mode switching operation, an imaging and storing operation, a forceps operation, an air/water supply operation, a suction operation, an electric scalpel operation, or the like, an angle knob 12 that is provided adjacent to the endoscope operating part 11, and a universal cord 13 including a connector portion 13A that attachably and detachably connects the endoscope 1 to a connecting portion 4A of the light source device 4.

The endoscope operating part 11 is provided with a treatment tool inlet port 112 into which a treatment tool for sampling a biological tissue such as a cell or a polyp is introduced. Although not shown in Fig. 1, inside the endoscope operating part 11 and the insertion part 10, various channels, such as a treatment tool channel, an air/water supply channel, and a suction channel, through which the treatment tool introduced from the treatment tool inlet port 112 is inserted are provided. The endoscope operating part 11 includes a suction button 11A, an air/water supply button 11B, and the like.

The insertion part 10 is composed of a soft part 10A having flexibility, a bendable part 10B provided at a distal end of the soft part 10A, and a distal end part 10C that is provided at a distal end of the bendable part 10B and that is harder than the soft part 10A. An imaging element and an imaging optical system are built in the distal end part 10C. The soft part 10A constitutes a flexible part between the bendable part 10B and the endoscope operating part 11 in the insertion part 10.

The bendable part 10B is configured to be bendable by a rotational movement operation of the angle knob 12. The bendable part 10B can be bent in any direction and at any angle depending on a site of the subject or the like for which the endoscope 1 is used, and the distal end part 10C can be directed in a desired direction.

A light guide configured by bundling a plurality of optical fibers is provided inside the endoscope 1, extending from the distal end part 10C of the insertion part 10 to the connector portion 13A. Light generated by the light source device 4 is introduced into the light guide from the connector portion 13A, advances to the distal end part 10C, and is emitted to the subject from an illumination window provided at the distal end part 10C.

The universal cord 13 includes the suction channel. For example, a suction device is provided in or externally attached to the light source device 4, and the suction device and the suction channel are connected to each other. In a case in which the suction button 11A included in the endoscope operating part 11 is operated, a negative pressure generated by the suction device is connected to a distal end of the endoscope 1, and a fluid or the like is sucked from the distal end of the endoscope 1 into the suction channel and is collected by the suction device.

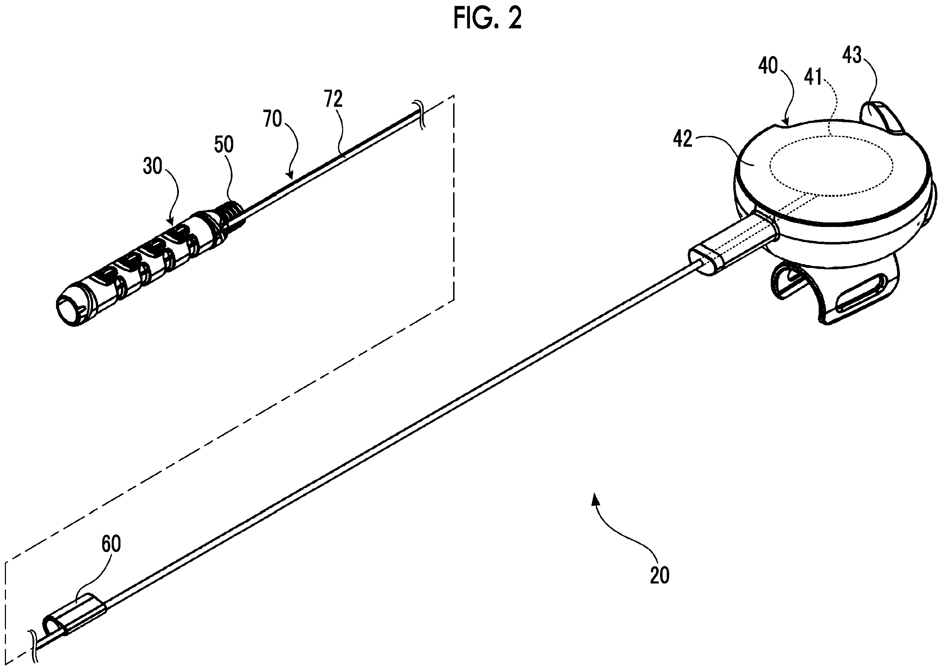

Fig. 2 is a perspective view showing an auxiliary device 20 for the endoscope 1 shown in Fig. 1. The auxiliary device 20 is configured to be attachable to and detachable from the endoscope 1, and can increase a movement range of the distal end part 10C in a case in which the bendable part 10B is bent, as compared with a case in which the auxiliary device 20 is not mounted on the endoscope 1. The auxiliary device 20 constitutes an endoscope auxiliary tool.

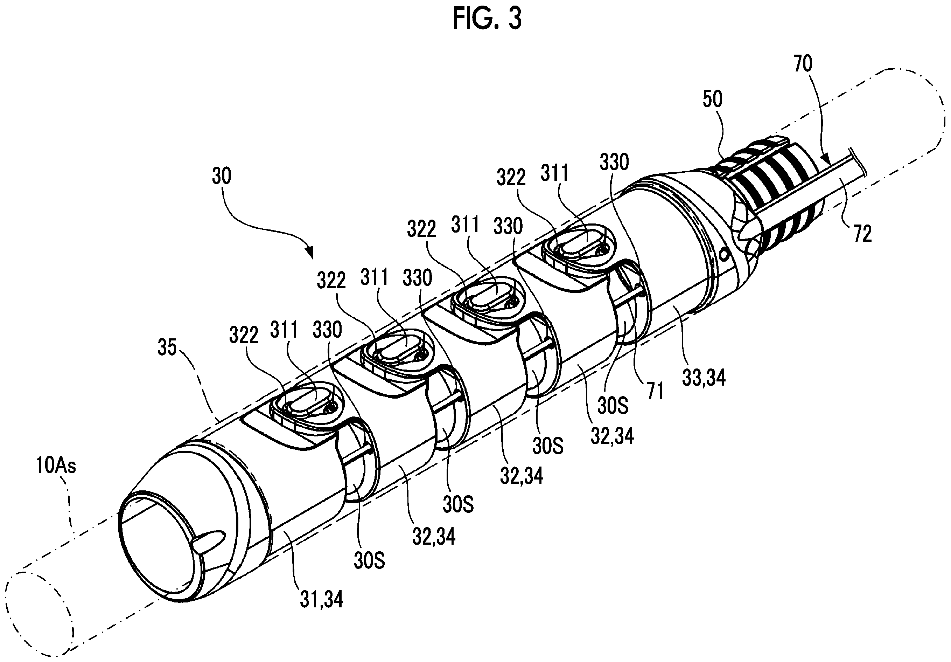

The auxiliary device 20 comprises a cylindrical bendable body 30 that is configured to cover and bend an end part 10As (see Fig. 3) on the bendable part 10B side of the soft part 10A and that is attachable to and detachable from the end part 10As, a support member 50 that is configured to support an end part on a base end side in an axial direction of the bendable body 30 (an end part on the endoscope operating part 11 side in a state where the bendable body 30 covers the end part 10As of the soft part 10A by being mounted on the end part 10As) at the end part 10As and that is configured separately from the bendable body 30, a first operating member 70 that is connected to the bendable body 30 and that is capable of bending the bendable body 30, an operating device 40 that is connected to the first operating member 70, and at least one locking member 60 that is provided on the first operating member 70 and that can be locked to the soft part 10A.

The operating device 40 comprises a rotational movement member 41 that is connected to a base end part of the first operating member 70 and that is rotationally movable, an accommodating member 42 that accommodates the rotational movement member 41, and a second operating member 43 that is provided to protrude from the accommodating member 42 and that is connected to the rotational movement member 41.

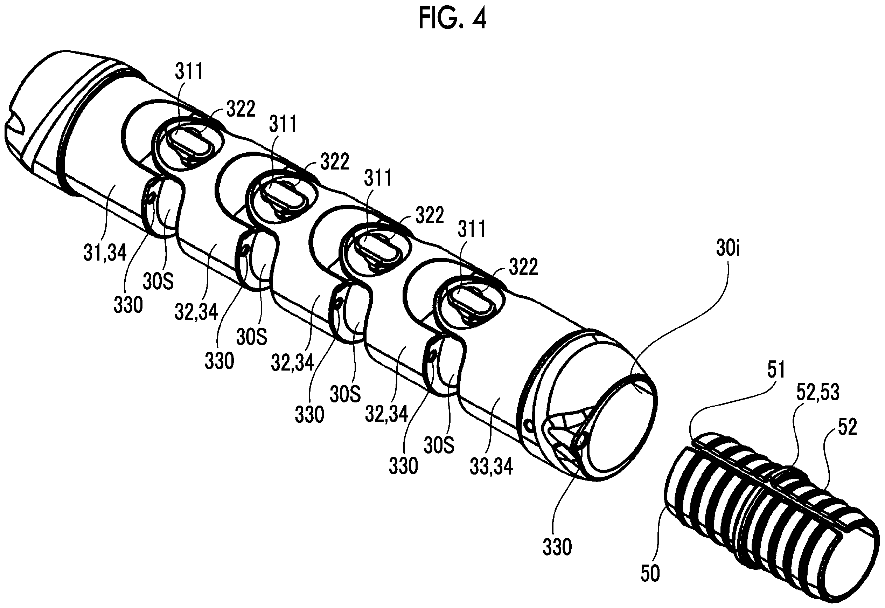

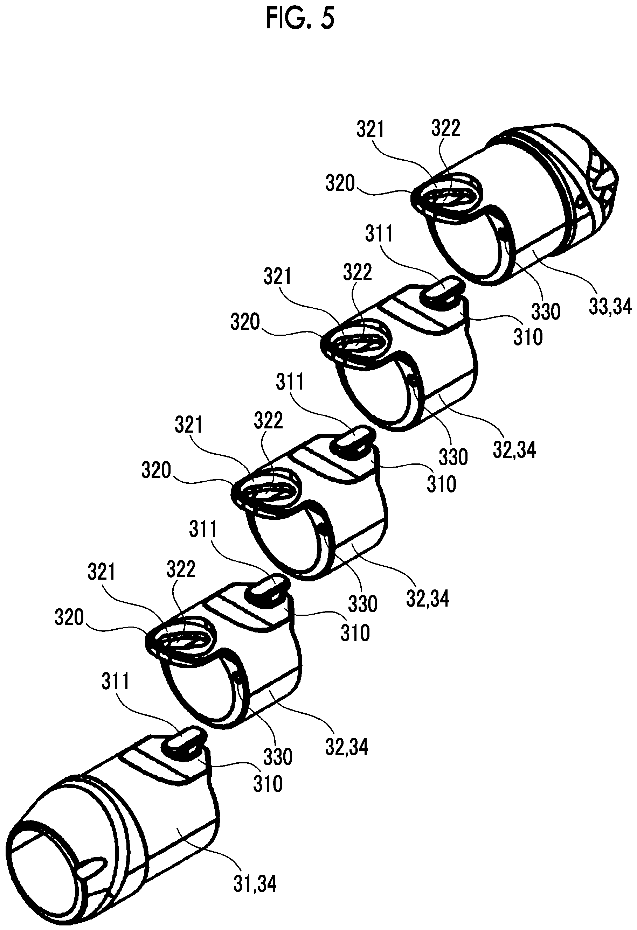

Fig. 3 is an enlarged view of the vicinity of the bendable body 30 and the support member 50 in the auxiliary device 20 shown in Fig. 2. Fig. 4 is a perspective view showing a state where the bendable body 30 and the support member 50 are separated from each other. Fig. 5 is an exploded perspective view of the bendable body 30.

As shown in Fig. 3, the bendable body 30 has an outer shape that is, for example, substantially cylindrical. An inner diameter of the bendable body 30 is larger than an outer diameter of the insertion part 10, making it possible to insert the insertion part 10 into the bendable body 30 and move the bendable body 30 to any position in the axial direction of the insertion part 10.

The bendable body 30 comprises a plurality of cylindrical nodal portions 34 (in the example of Fig. 3, a total of five nodal portions, including a distal end nodal portion 31 on a distal end side, a base end nodal portion 33 on the base end side, and three intermediate nodal portions 32 between the distal end nodal portion 31 and the base end nodal portion 33) arranged in the axial direction, and a cylindrical coating member 35. Each of the plurality of nodal portions 34 supports an adjacent nodal portion 34 to be rotationally movable.

As shown in Fig. 5, each of four nodal portions 34 excluding the base end nodal portion 33 among the five nodal portions 34 is provided with a protruding piece 310 that protrudes from a part of a base end edge to the base end side. The protruding piece 310 is provided with an engagement protrusion 311 that protrudes in a radial direction of the nodal portion 34. The engagement protrusion 311 includes a columnar portion that protrudes from the protruding piece 310 and an oval portion that is connected to the columnar portion. An outer diameter of the columnar portion is equal to or smaller than a length of the oval portion in a minor axis direction. A major axis direction of the oval portion of the engagement protrusion 311 coincides with an axial direction of the nodal portion 34.

Each of four nodal portions 34 excluding the distal end nodal portion 31 among the five nodal portions 34 is provided with a protruding piece 320 that protrudes from a part of a distal end edge to the distal end side. The protruding piece 320 is provided with a recess portion 321 which is substantially circular as viewed in the radial direction of the nodal portion 34, and the recess portion 321 is provided with a through-hole 322 that penetrates the nodal portion 34 in the radial direction. The through-hole 322 has an oval shape so that the oval portion of the engagement protrusion 311 can be inserted therethrough. A major axis direction of the through-hole 322 intersects the axial direction of the nodal portion 34.

Focusing on two adjacent nodal portions 34 among the five nodal portions 34, the engagement protrusion 311 of one of the two nodal portions 34 is inserted into the through-hole 322 of the other of the two nodal portions 34, and the engagement protrusion 311 engages with the through-hole 322 and the recess portion 321 in which the through-hole 322 is formed by being held in a state where the major axis direction of the engagement protrusion 311 and the major axis direction of the through-hole 322 intersect with each other. In this state, as shown in Figs. 3 and 4, a slit 30S as a gap is formed between the two adjacent nodal portions 34. Since the columnar portion of the engagement protrusion 311 is located inside the through-hole 322 and the oval portion of the engagement protrusion 311 is located in the recess portion 321, the engagement protrusion 311 is rotationally movable around an axis extending in a protruding direction of the engagement protrusion 311 in the recess portion 321.

The recess portion 321 in the nodal portion 34 and the through-hole 322 provided in the recess portion 321 constitute an engagement hole portion that engages with the engagement protrusion 311 of the nodal portion 34 adjacent to the nodal portion 34. As described above, the two adjacent nodal portions 34 are supported by each other so as to be relatively rotationally movable through the engagement between the engagement hole portion provided in one nodal portion 34 and the engagement protrusion provided in the other nodal portion 34.

As shown in Fig. 3, the first operating member 70 comprises an elongated wire 71 and a guide member 72 through which the wire 71 is inserted. The guide member 72 is composed of, for example, a compression coil spring, or a compression coil spring and a coating member that covers the compression coil spring.

As shown in Figs. 3 and 4, each of the five nodal portions 34 in the bendable body 30 is provided with a hole portion 330 through which the wire 71 can be inserted. As shown in Fig. 3, the wire 71 is inserted in the order of the hole portion 330 of the base end nodal portion 33, the hole portion 330 of the intermediate nodal portion 32, the hole portion 330 of the intermediate nodal portion 32, the hole portion 330 of the intermediate nodal portion 32, and the hole portion 330 of the distal end nodal portion 31, and a distal end of the wire 71 is supported by the distal end nodal portion 31. A base end of the wire 71 is connected to the rotational movement member 41 of the operating device 40. As the rotational movement member 41 rotates, the wire 71 moves in an axial direction thereof. The two adjacent nodal portions 34 approach or separate from each other in a range of a width of the slit 30S due to the movement of the wire 71. As a result, the bendable body 30 can take a plurality of bent shapes.

The coating member 35 covers a connecting portion between the two adjacent nodal portions 34 so as not to damage an organ or the like of the subject due to the approach or the separation of the two adjacent nodal portions 34 and not to inhibit the approach of the nodal portions 34. It is preferable that the coating member 35 is made of a flexible material such as rubber so as to be able to follow the bending operation of the five nodal portions 34. It is preferable that the nodal portion 34 is made of a highly rigid material such as metal or resin so that a bending force during bending can be efficiently transmitted to the end part 10As. From the viewpoint of ease of forming, weight reduction, and manufacturing cost, it is preferable that the nodal portion 34 is mainly made of resin. As described above, the bendable body 30 is configured such that the five nodal portions 34 are connected to each other only by an engagement force. Therefore, it is possible to facilitate the manufacturing of the bendable body 30 and to reduce the manufacturing cost.

As shown in Fig. 2, the guide member 72 is provided between the base end nodal portion 33 and the operating device 40, and has a cylindrical shape through which the wire 71 is inserted. A distance from the bendable body 30 to the operating device 40 is large and is close to a length of the insertion part 10 of the endoscope 1. Therefore, between the bendable body 30 and the operating device 40, the guide member 72 guides the movement of the wire 71 in the axial direction, so that the wire 71 can be easily pulled at a position away from the bendable body 30.

As shown in Fig. 2, the first operating member 70 is exposed between the bendable body 30 and the operating device 40. An exposed portion of the first operating member 70 is provided with the locking members 60 at a plurality of positions. The locking member 60 is composed of a clip or the like having a C-shaped cross section that can be locked to the insertion part 10 of the endoscope 1. By locking the locking member 60 to the insertion part 10 at a plurality of positions in the axial direction of the insertion part 10, the first operating member 70 can be maintained aligned along the insertion part 10 in a state where the auxiliary device 20 is in use.

As described above, the bendable body 30 can be moved in the axial direction of the insertion part 10 along an outer peripheral surface of the insertion part 10. In a case in which the auxiliary device 20 is used, it is necessary to hold the position of the bendable body 30 in the axial direction of the insertion part 10 at a desired position. In the present embodiment, the support member 50 is provided to hold the position of the bendable body 30.

As shown in Fig. 4, the support member 50 has a cylindrical shape that can cover an outer peripheral surface of the end part 10As of the insertion part 10. The outer peripheral surface of the end part 10As and an inner peripheral surface of the support member 50 can be in close contact with each other. The support member 50 has flexibility, and a linear slit 51 extending in the axial direction is provided on an outer peripheral surface thereof. The support member 50 can be easily mounted at any position in the axial direction of the insertion part 10 by widening the slit 51 and covering the outer peripheral surface of the insertion part 10 in the radial direction of the insertion part 10.

A plurality of (nine in the example in Fig. 4) C-shaped protrusions 52 that extend along a circumferential direction are arranged on an outer peripheral surface of the support member 50 in the axial direction. Among the nine protrusions 52, a central protrusion 53 in the middle has a protrusion height higher than the other eight protrusions 52. Outer diameters of the eight protrusions 52 excluding the central protrusion 53 among the nine protrusions 52 are the same as or slightly larger than the inner diameter of the bendable body 30. An outer diameter of the central protrusion 53 is larger than the inner diameter of the bendable body 30. The central protrusion 53 constitutes a first protrusion, and the protrusions 52 other than the central protrusion 53 constitute a second protrusion.

A flow of mounting the bendable body 30 on the end part 10As is as follows. First, the support member 50 is brought into close contact with the outer peripheral surface of the end part 10As by covering the outer peripheral surface. It is preferable that the support member 50 is made of a material having a sufficiently large frictional force with the outer peripheral surface of the insertion part 10. In this way, the position of the support member 50 can be firmly held in a state where the support member 50 covers the outer peripheral surface of the end part 10As.

Next, the insertion part 10 of the endoscope 1 is inserted into the bendable body 30 from the distal end part 10C side, and the bendable body 30 is moved to the front of the support member 50 along the insertion part 10. Next, the bendable body 30 is further moved to the base end side, and the bendable body 30 is pushed to the base end side such that the support member 50 that is in close contact with the end part 10As is inserted into the base end nodal portion 33. Since the outer diameter of the central protrusion 53 of the support member 50 is large, as the bendable body 30 is pushed to the base end side, a base end surface of the base end nodal portion 33 abuts against the central protrusion 53, thereby restricting further pushing of the bendable body 30 to the base end side.

Through the above steps, an inner peripheral surface of the base end nodal portion 33 of the bendable body 30 and the plurality of protrusions 52 of the support member 50 are brought into pressure contact with each other. The protrusion 52 extends along the circumferential direction of the inner peripheral surface of the base end nodal portion 33. Therefore, in this state, the rotation of the bendable body 30 about its axis is firmly suppressed by a frictional force between the base end nodal portion 33 and the protrusion 52. In addition, since the plurality of protrusions 52 aligned in the axial direction of the bendable body 30 are brought into pressure contact with the inner peripheral surface of the base end nodal portion 33, the movement of the bendable body 30 in the axial direction is also firmly suppressed by a frictional force between the base end nodal portion 33 and the plurality of protrusions 52. In this way, a base end (specifically, the base end nodal portion 33) of the bendable body 30 in the axial direction is supported at the end part 10As. As described above, the support member 50 functions to position the bendable body 30 in the axial direction of the insertion part 10 and to position the bendable body 30 in the circumferential direction of the insertion part 10.

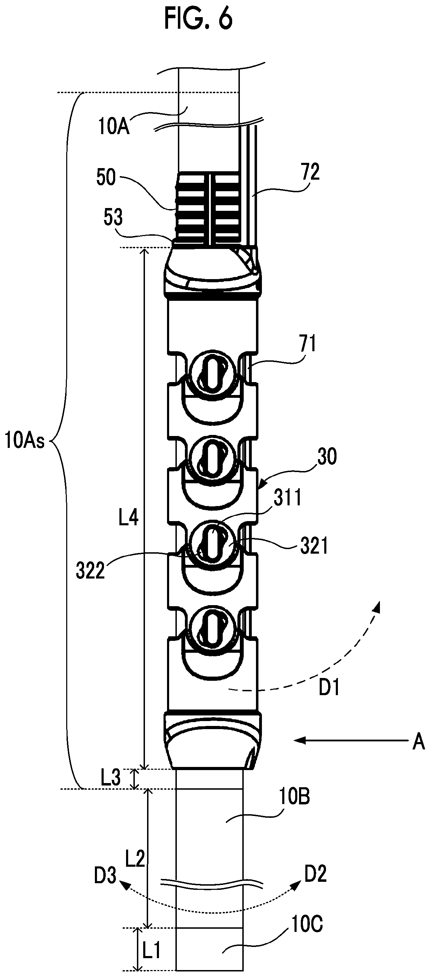

Fig. 6 is a diagram showing a state where the bendable body 30 is supported by the support member 50 at the end part 10As. Fig. 6 shows a state of the bendable body 30 as viewed in a direction of a rotational movement axis of the nodal portion 34 in the bendable body 30. As shown in Fig. 6, in a case in which the auxiliary device 20 is used, in a state where both ends of the bendable body 30 in the axial direction are located closer to the soft part 10A side than the bendable part 10B, a base end that is one of both ends of the bendable body 30 is supported by the support member 50 at the end part 10As.

In a case in which the wire 71 is pulled to an upper side in the drawing in the state shown in Fig. 6, five nodal portions 34 are rotationally moved counterclockwise in the drawing in order from the distal end side. In this way, the bendable body 30 can be bent in a direction D1 in the drawing. In a case in which the bendable body 30 is bent in the direction D1, the end part 10As supporting the bendable body 30 is bent in the direction D1 following the bending.

The bendable part 10B can be bent in a clockwise direction D3 and a counterclockwise direction D2 in Fig. 6 by operating the angle knob 12. The bendable body 30 can also be supported at the end part 10As in a state where the distal end of the bendable body 30 is located at the bendable part 10B. However, as shown in Fig. 6, since both ends of the bendable body 30 are located at the end part 10As, the bendable part 10B can be bent to the maximum extent. In a case in which the bendable body 30 is bent, in addition to the bending of the distal end part 10C due to the bending of the bendable part 10B, the distal end part 10C can be bent by the bending of the end part 10As. As described above, by using the auxiliary device 20, the bending state of the distal end part 10C can be flexibly changed as compared with a case in which the auxiliary device 20 is not used.

Fig. 6 shows a length L1 of the distal end part 10C in the axial direction of the insertion part 10, a length L2 of the bendable part 10B in the axial direction of the insertion part 10, a length L4 of the bendable body 30 in the axial direction, and a distance L3 between the distal end of the bendable body 30 and the bendable part 10B.

In consideration of the actual use environment and operability of the endoscope 1, it is preferable that the length L4 is approximately equal to a value obtained by adding up the length L1, the length L2, and the distance L3. In this way, it is possible to easily direct the distal end part 10C toward a treatment target site that is located in a recessed area, for example. In addition, the orientation of the distal end part 10C can be easily and stably adjusted. In addition, since the bendable body 30 has an appropriate length, it is possible to reduce the manufacturing cost and to improve the mountability on the end part 10As.

In a case in which the length L 1 is, for example, 17 mm and the length L2 is, for example, 53 mm, the distance L3 can be set to, for example, 8 mm and the length L4 can be set to, for example, 74.2 mm, but the present invention is not limited to this.

As the value of the distance L3 becomes larger, the distal end part 10C can be bent to a greater extent. Note that, in consideration of the actual use environment of the endoscope 1, it is sufficient to set the maximum value of the distance L3 to about the length L4.

Generally, the outer peripheral surface of the insertion part 10 of the endoscope 1 is marked with a scale. The bendable body 30 may be capable of being positioned in the axial direction of the insertion part 10 with reference to the scale.

For example, the central protrusion 53 of the support member 50 is disposed to overlap the scale, and the bendable body 30 is pushed into the support member 50, so that the bendable body 30 can be mounted at an appropriate position. In this way, a surgeon can determine the mounting position of the bendable body 30 without hesitation and can perform endoscopy efficiently.

Considering the actual use environment of the auxiliary device 20, it is preferable that the direction D1 and the direction D2 shown in Fig. 6 are substantially the same. In a case in which the direction D1 and the direction D2 are the same, the distal end part 10C can be bent to a greater extent.

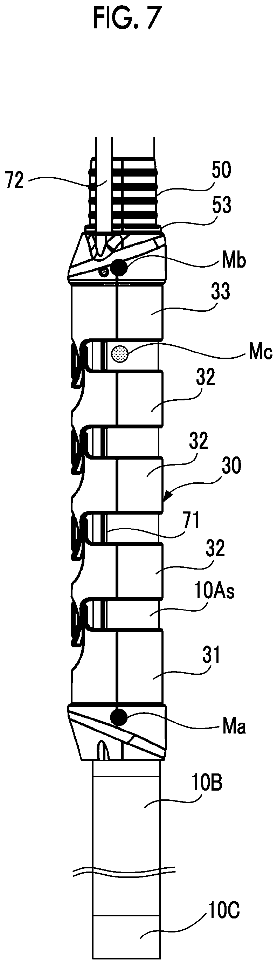

Fig. 7 is a side view of the endoscope 1 and the auxiliary device 20 in Fig. 6 as viewed in a direction of an arrow A. As shown in Fig. 7, it is preferable that the bendable body 30 is provided with a mark Ma that serves as a guide for matching the direction D1 and the direction D2. In addition, it is preferable that the bendable body 30 is provided with a mark Mb that serves as a guide for positioning the bendable body 30 in the circumferential direction of the insertion part 10. In addition, it is preferable that the end part 10As of the soft part 10A is provided with a mark Mc that serves as a guide for positioning the bendable body 30 in the circumferential direction of the insertion part 10.

The mark Ma is provided on an outer peripheral surface of the distal end nodal portion 31. The mark Mb is provided on an outer peripheral surface of the base end nodal portion 33. In the example of Fig. 7, the mark Ma and the mark Mb are provided on a side surface of the bendable body 30 in a bendable direction. In addition, the mark Mc is provided on a side surface on a side in the direction D2, which is a bending direction of the bendable part 10B.

In a case of determining a rotational position of the bendable body 30, the orientation of the bendable body 30 with respect to the soft part 10A is adjusted such that the mark Mb and the mark Mc are substantially at the same position in the circumferential direction of the soft part 10A. In this way, the rotational position of the bendable body 30 can be provisionally determined.

After the bendable body 30 is pushed into the support member 50, the angle knob 12 is operated to bend the bendable part 10B in the direction D2 (front side of the paper surface in Fig. 7). Then, the rotational position of the bendable body 30 is adjusted such that the distal end part 10C and the mark Ma at that time are aligned on a straight line. In this way, by providing the marks on the bendable body 30 and the soft part 10A, the bendable body 30 can be mounted on the soft part 10A in an appropriate orientation.

Even in a case in which the mark Ma and the mark Mb are not provided on the bendable body 30, the rotational position of the bendable body 30 can be provisionally determined by aligning a distal end of the guide member 72 extending from the base end nodal portion 33 with the mark Mc, and then the rotational position of the bendable body 30 is adjusted by bending the bendable part 10B such that the distal end part 10C and the distal end of the guide member 72 are aligned on a straight line, so that the direction D1 and the direction D2 can match each other.

In the above description, the base end of the bendable body 30 is supported by the support member 50 at the end part 10As, but the present invention is not limited to this. The distal end of the bendable body 30 can also be supported by the support member 50 at the end part 10As.

In this case, first, in a state where the insertion part 10 is covered with the bendable body 30 from the distal end part 10C side, the bendable body 30 is moved to the base end side. Thereafter, the support member 50 is mounted near the bendable part 10B at the end part 10As so as not to overlap the bendable part 10B. Thereafter, the bendable body 30 is moved to the distal end side and slightly moved back, and the distal end nodal portion 31 is pushed into the support member 50. In this way, the distal end of the bendable body 30 can be supported at the end part 10As.

As described above, with the configuration in which the base end of the bendable body 30 is supported by the support member 50 at the end part 10As, the bendable body 30 only needs to be moved in one direction. Therefore, the mountability of the bendable body 30 can be improved.

The support member 50 has a line-symmetrical shape as viewed in the radial direction, with the central protrusion 53 as a boundary line. In this way, regardless of whether the base end side of the bendable body 30 is supported at the end part 10As or the distal end side of the bendable body 30 is supported at the end part 10As, it is not necessary to be conscious of an attachment direction of the support member 50 to the insertion part 10. Therefore, the endoscopy can be efficiently performed.

In the auxiliary device 20, a configuration may be adopted in which two support members 50 are provided and both ends of the bendable body 30 are supported by the respective support members 50 at the end part 10As.

The bendable body 30 has a configuration in which a plurality of nodal portions 34 are each independently provided and are rotationally movably connected to each other, but the present invention is not limited to this. For example, a structure in which the plurality of nodal portions 34 are integrally formed may be achieved by performing laser processing on one pipe. In this way, the manufacturing cost can be reduced.

In addition, in a case of the structure in which the plurality of nodal portions 34 are integrally formed as described above, a slit extending in the axial direction may be provided on an outer peripheral surface of the bendable body 30. In this manner, the slit can be opened, and the bendable body 30 can be mounted at the end part 10As in the radial direction of the soft part 10A, making it possible to easily mount the bendable body 30.

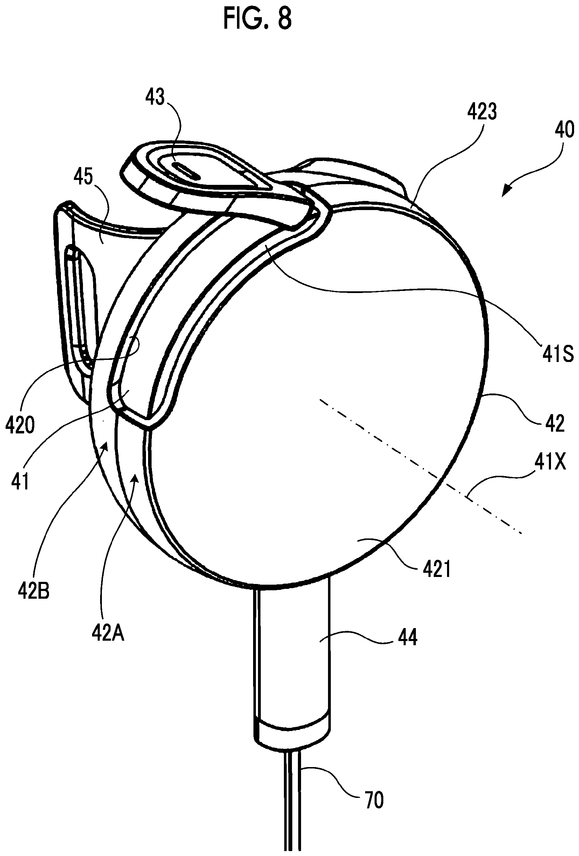

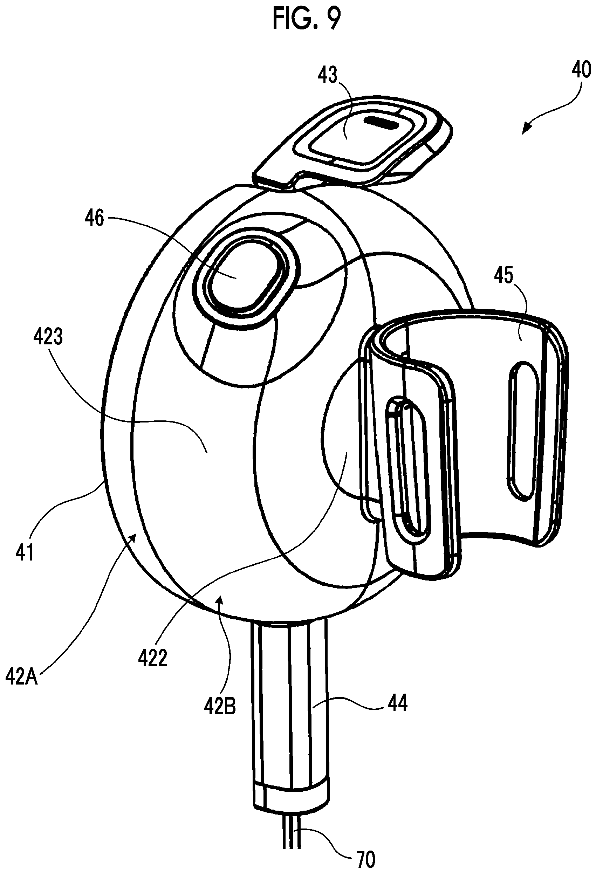

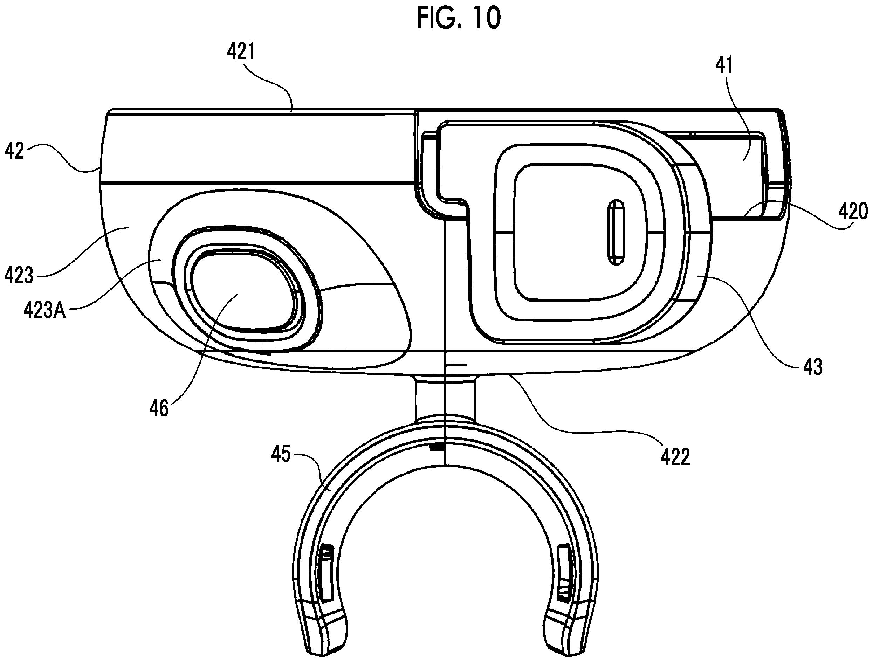

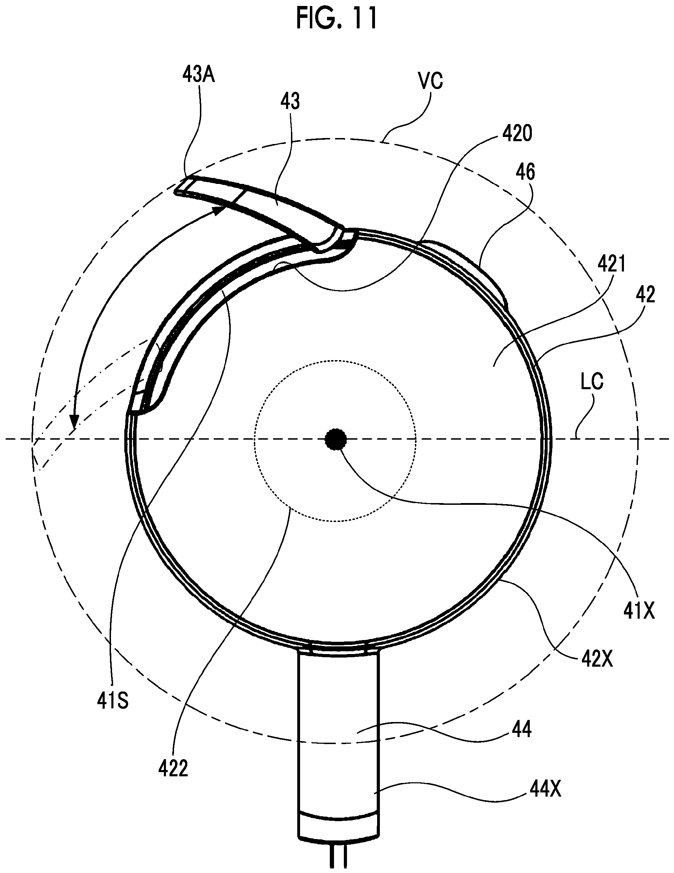

Fig. 8 is a perspective view of the operating device 40 as viewed from a front side. Fig. 9 is a perspective view of the operating device 40 as viewed from a rear side. Fig. 10 is a plan view of the operating device 40. Fig. 11 is a front view of the operating device 40.

The operating device 40 comprises the rotational movement member 41 connected to the first operating member 70, the accommodating member 42 that accommodates the rotational movement member 41, the second operating member 43 that protrudes from the accommodating member 42, a reinforcing member 44 that reinforces a base end side of the first operating member 70 located outside the accommodating member 42, and a locking part 45 that can be locked to the endoscope 1. The operating device 40 can rotationally move the rotational movement member 41 connected to the second operating member 43 by rotationally moving the second operating member 43 (rotationally moving the second operating member 43 counterclockwise as viewed from a front surface of Fig. 11) and can pull (pull to the base end side) the wire 71 connected to the rotational movement member 41.

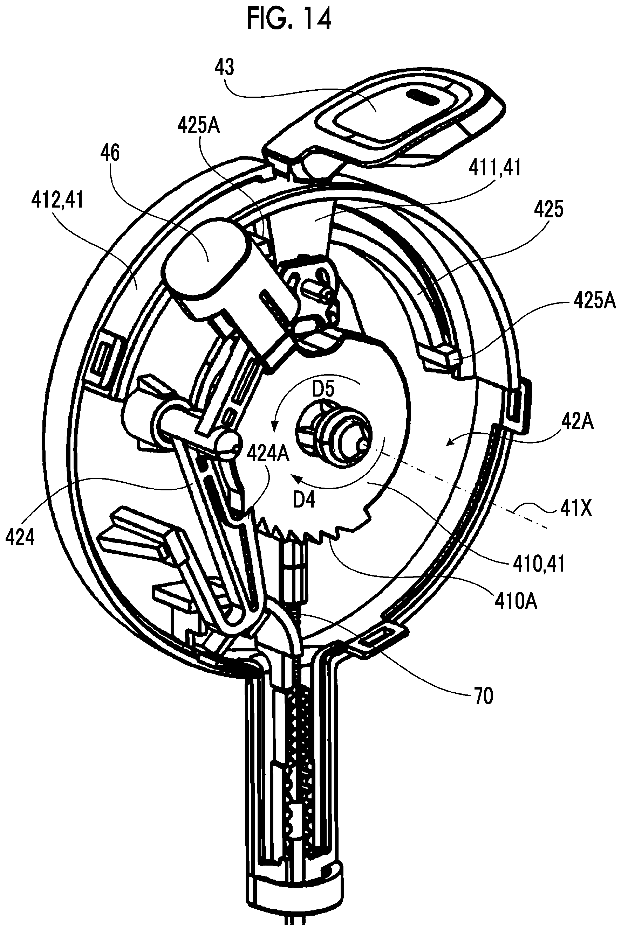

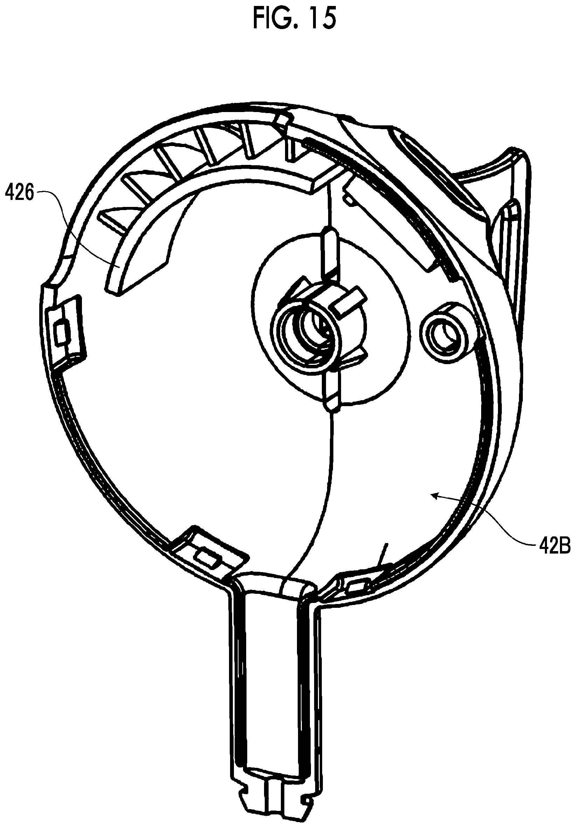

The accommodating member 42 and the reinforcing member 44 comprise a front cover member 42A and a rear cover member 42B connected to the front cover member 42A, and are configured to be connected to each other. Fig. 14 is a diagram showing a state where the rear cover member 42B in Fig. 9 is removed. Fig. 15 is a perspective view showing an inside of the rear cover member 42B. As shown in Figs. 14 and 15, an accommodation space in which the rotational movement member 41, the first operating member 70, and the like can be accommodated is formed between the front cover member 42A and the rear cover member 42B.

The front cover member 42A and the rear cover member 42B are each composed of a circular portion 42X and a rectangular portion 44X having a substantially rectangular shape extending from an outer peripheral edge of the circular portion 42X in a radial direction of the circular portion 42X, as shown in Fig. 11, as viewed in a direction of a rotational movement axis 41X of the rotational movement member 41. The circular portion 42X of the front cover member 42A and the circular portion 42X of the rear cover member 42B are connected to each other to form a substantially disk-shaped accommodating member 42, and the rectangular portion 44X of the front cover member 42A and the rectangular portion 44X of the rear cover member 42B are connected to each other to form a substantially elliptical columnar reinforcing member 44.

The accommodating member 42 has, as its outer surfaces, a first surface 421 (see Fig. 8) on one end side (front side) in the direction of the rotational movement axis 41X of the rotational movement member 41, a second surface 422 (see Fig. 9) on the other end side (rear side) in the direction of the rotational movement axis 41X, and a third surface 423 (see Figs. 8 and 9) connecting the first surface 421 and the second surface 422. The first surface 421 and the second surface 422 are each formed of, for example, a circular flat surface perpendicular to the rotational movement axis 41X. As shown in Fig. 11, as viewed in the direction of the rotational movement axis 41X, the first surface 421 is configured to be larger than the second surface 422, and the second surface 422 is located substantially at the center of the first surface 421. The third surface 423 is configured as a curved surface that curves from an outer peripheral edge of the first surface 421 toward an outer peripheral edge of the second surface 422.

As shown in Figs. 9 and 10, the second surface 422 is provided with the locking part 45 having a C-shaped cross section. The locking part 45 is configured to be locked to, for example, a distal end part 113 (see Fig. 1) of the endoscope operating part 11. By locking the locking part 45 to the distal end part 113, the operation of the operating device 40 can be stably performed.

As shown in Figs. 8 and 10, an opening portion 420 is provided in the third surface 423 of the accommodating member 42. A part of the rotational movement member 41 (a bending member 412 (see Fig. 14) to be described below) is exposed from the opening portion 420. The second operating member 43 is provided on the exposed portion of the rotational movement member 41. As described above, the second operating member 43 is configured to protrude from the accommodating member 42 via the opening portion 420 and is movable in the opening portion 420. The opening portion 420 is formed to extend from the third surface 423 to the first surface 421. Therefore, as shown in Figs. 8 and 11, a front region 41S on the front side of the exposed portion of the rotational movement member 41 is visible from the front side.

Fig. 11 shows a virtual circle VC centered on the rotational movement axis 41X of the rotational movement member 41. As shown in Fig. 11, an outer peripheral edge of the accommodating member 42 has a shape (preferably a circular shape) along the virtual circle VC as viewed in the direction of the rotational movement axis 41X. A protruding end 43A of the second operating member 43 is configured to be movable along a portion of a circumference of the virtual circle VC. A bidirectional arrow in the drawing indicates a movement range of the second operating member 43.

As shown in Fig. 11, the second operating member 43 has a curved shape as viewed in the direction of the rotational movement axis 41X. The second operating member 43 can have a simple flat plate shape, and can have a curved shape to improve the operability of the second operating member 43. The second operating member 43 can be configured to extend along a radial direction of the virtual circle VC, but in the present embodiment, the second operating member 43 is configured to extend in a direction intersecting the radial direction of the virtual circle VC. As described above, a protruding angle of the second operating member 43 with respect to the accommodating member 42 is an acute angle, so that the operability of the second operating member 43 can be improved.

In the present embodiment, the opening portion 420 is provided in a range of about 1/4 of the outer peripheral edge of the accommodating member 42, and the second operating member 43 is configured to be movable in this range. As will be described below, the second operating member 43 is operated by a thumb. Therefore, within this range, the second operating member 43 can be easily rotationally moved with the thumb.

The reinforcing member 44 is provided to protrude on a side opposite to the second operating member 43 with the rotational movement axis 41X interposed therebetween on the third surface 423 of the accommodating member 42. Fig. 11 shows a straight line LC that divides the virtual circle VC into two regions. In a case in which one of the two divided regions of the virtual circle VC is a first region and the other is a second region, the second operating member 43 and the opening portion 420 are provided in the first region, and the reinforcing member 44 is provided in the second region. The entire accommodating member 42 is located inside the virtual circle VC as viewed in the direction of the rotational movement axis 41X.

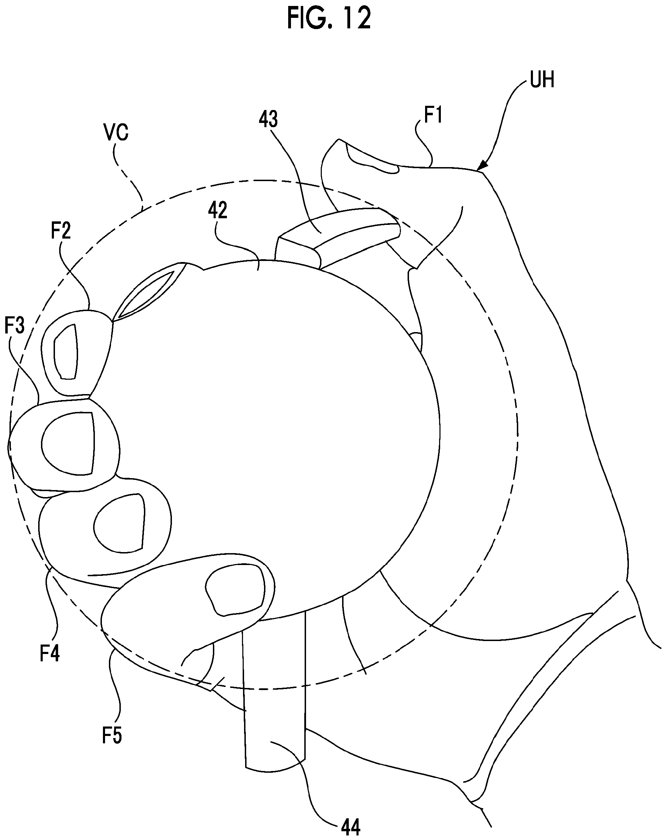

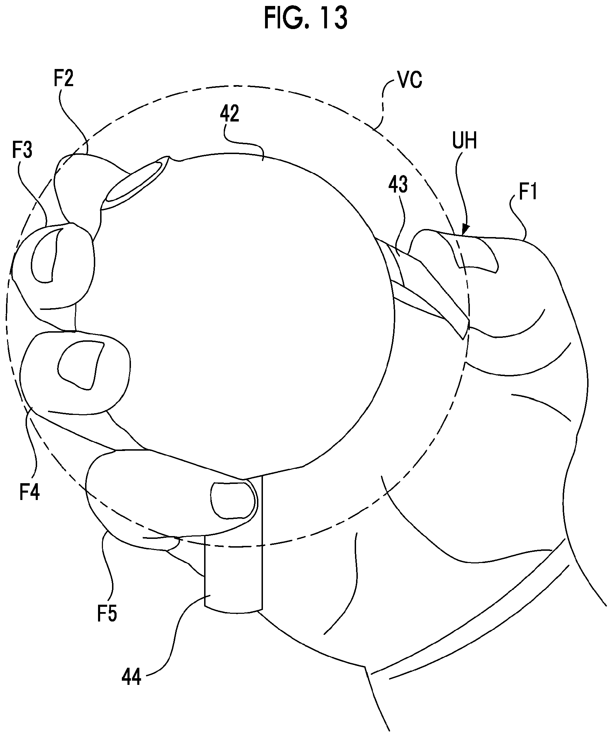

Figs. 12 and 13 are schematic diagrams showing an example of an operation method of the operating device 40. Figs. 12 and 13 are views of the operating device 40 as viewed from the rear side. As shown in Fig. 12, an operator grips the accommodating member 42 with four fingers (an index finger F2, a middle finger F3, a ring finger F4, and a little finger F5) other than a thumb F1 of one hand UH in a state where the thumb F1 is placed on the second operating member 43. In this state, a part of a palm of the one hand UH can contact the first surface 421 of the accommodating member 42, but this contact is not essential.

From the state shown in Fig. 12, in a case in which the operator moves the thumb F1 clockwise in the drawing along the outer peripheral edge of the accommodating member 42, the second operating member 43 is rotationally moved as shown in Fig. 13, and the wire 71 is pulled. In Fig. 13, the little finger F5 is moved to the position of the reinforcing member 44 in order to stabilize the gripping of the accommodating member 42 during the movement of the second operating member 43, but such finger movement is optional.

As shown in Fig. 13, the operator can also grip the accommodating member 42 with three fingers (the index finger F2, the middle finger F3, and the ring finger F4) other than the thumb F1 of the one hand UH in a state where the thumb F1 is placed on the second operating member 43. As described above, the accommodating member 42 is configured to have, on the inside of the virtual circle VC, a region that can be gripped by three or four fingers other than the thumb F1, which is placed on the second operating member 43, of the one hand UH of the operator.

In order to enable the second operating member 43 to be rotationally moved with a strong force, it is important that a distance between the movable thumb F1 that is in contact with the second operating member 43 and the other three or four fingers gripping the accommodating member 42 does not become far regardless of the movement position of the thumb F1. According to the present embodiment, the fingers gripping the accommodating member 42 are present inside the rotational movement circle (in other words, the virtual circle VC) in a case in which the thumb F1 moves. Therefore, the second operating member 43 can be rotationally moved with a strong force.

In addition, with the operating device 40, the accommodating member 42 has a disk shape that fits into the virtual circle VC, so that the accommodating member 42 can be easily fitted in the hand in a state where the accommodating member 42 is gripped, and the operability can be improved. In addition, since the accommodating member 42 has a disk shape, the same gripping state is achieved regardless of the gripping position. Therefore, the accommodating member 42 can be gripped at a position where the operator can most easily move the thumb with respect to the position of the second operating member 43. In addition, since the center of the accommodating member 42 and the rotational movement axis 41X are coincident, the rotational movement axis 41X is held at the center of the palm of the operator, making it easier for the operator to reach the second operating member 43 with his or her thumb. In this holding position, the thumb can be largely moved back and forth with a base of the thumb as a base point, so that an effect of easily rotationally moving the second operating member 43 can be obtained.

With the operating device 40, the first surface 421 of the accommodating member 42 is formed of a flat surface, and the third surface 423 is formed of a curved surface from the first surface 421 toward the second surface 422. Therefore, as shown in Figs. 12 and 13, in a case in which the accommodating member 42 is gripped with fingers other than the thumb F1, the fingers can be pressed into an edge of the first surface 421 of the accommodating member 42, enabling stable gripping. In addition, since the third surface 423 is formed of a curved surface, a pad of the finger can be fitted to the curved surface, enabling stable gripping.

With the operating device 40, the reinforcing member 44 is provided to protrude on a side of the accommodating member 42 opposite to the second operating member 43 on which the thumb F1 is disposed. Therefore, as shown in Fig. 13, the reinforcing member 44 can function as a finger hook portion for hooking the little finger. As a result, the rotational movement operation of the second operating member 43 can be stably performed while reinforcing the base end part of the first operating member 70.

As shown in Fig. 14, the rotational movement member 41 is composed of a substantially disk-shaped rotational movement axis member 410 having a plurality of ratchet grooves 410A formed on its outer peripheral surface, a flat plate-shaped protruding piece 411 that protrudes radially from the rotational movement axis member 410, and a bending member 412 that is connected to the protruding piece 411 and that is bent in an arc shape. The bending member 412 is provided with the second operating member 43. The wire 71 of the first operating member 70 is connected to the protruding piece 411.

A lock member 424 including a lock piece 424A that is engageable with the ratchet groove 410A is provided inside the front cover member 42A. In a case in which the rotational movement axis member 410 is rotationally moved in a direction D4 in Fig. 14 (in a case in which the state of Fig. 12 transitions to the state of Fig. 13), the lock piece 424A can climb over the ratchet groove 410A and engage with an adjacent ratchet groove 410A. On the other hand, in a state where the lock piece 424A and the ratchet groove 410A are engaged with each other, the lock piece 424A prevents the rotational movement axis member 410 from being rotationally moved in a direction D5 opposite to the direction D4. As described above, the lock member 424 and the ratchet grooves 410A form a lock mechanism that locks the rotational movement position of the rotational movement member 41 in a plurality of stages.

A lock release member 46 that is partially exposed from the accommodating member 42 is connected to the lock member 424. The above-described lock mechanism is capable of releasing the engagement between the lock piece 424A and the ratchet groove 410A by operating the lock release member 46 (pressing the lock release member 46 toward the inside of the accommodating member 42). Therefore, in a case in which the lock release member 46 is pressed, the rotational movement axis member 410 can be rotationally moved in the direction D5.

On the inside of the front cover member 42A, a bending rib 425 is provided on the rotational movement axis 41X side of the bending member 412. The bending rib 425 is provided to extend along the rotational movement direction of the rotational movement member 41. Column portions 425A that protrude toward the rear cover member 42B are provided at both ends of the bending rib 425.

As shown in Fig. 15, on the inside of the rear cover member 42B, a bending rib 426 is provided at a position facing the bending rib 425 of the front cover member 42A. The bending rib 426 is provided to extend along the rotational movement direction of the rotational movement member 41. Both end parts of the bending rib 426 abut against the column portion 425A of the bending rib 425.

The protruding piece 411 of the rotational movement member 41 is disposed between the bending rib 425 and the bending rib 426, and is movable in a space defined by the bending rib 425 and the bending rib 426. The protruding piece 411 is sandwiched between the bending rib 425 and the bending rib 426. The protruding piece 411 is sandwiched between the bending rib 425 and the bending rib 426 to such an extent that a predetermined frictional force is generated between the bending rib 425 and the bending rib 426.

In a case in which the wire 71 is pulled to the base end side, the second operating member 43 is rotationally moved in the direction D4 with a force exceeding a frictional force between the protruding piece 411 and the bending ribs 425 and 426, so that the rotational movement member 41 is rotationally moved in a stepwise manner, and the wire 71 can be pulled in a stepwise manner. In this manner, in a case in which the lock release member 46 is pressed in a state where the wire 71 is pulled to the base end side, the lock piece 424A is disengaged from the ratchet groove 410A. In this case, a restoring force acts on the rotational movement member 41 to return the wire 71 to its original position. The frictional force between the protruding piece 411 and the bending ribs 425 and 426 is set to be larger than the restoring force. Therefore, even in a case in which the lock release member 46 is pressed in a state where the wire 71 is pulled to the base end side, the rotational movement of the rotational movement member 41 in the direction D5 is suppressed by the restoring force of the wire 71. As described above, the bending rib 425 and the bending rib 426 constitute a movement suppression mechanism that suppresses the movement of the rotational movement member 41 to a reference position (position in a state where the wire 71 is not pulled) in a case in which the lock release member 46 is operated.

Since the movement suppression mechanism is provided, the bendable body 30 does not return to its original linear form only by operating the lock release member 46 in a state where the bendable body 30 of the auxiliary device 20 is bent. Since the bendable body 30 can be disposed in the body, safety for the subject can be improved by suppressing the change in the shape of the bendable body 30 through the operation of the lock release member 46.

In a case in which it is desired for the second operating member 43 to be returned from the state shown in Fig. 13 to the state shown in Fig. 12, the operation of rotationally moving the second operating member 43 in the direction D5 of Fig. 14 need only be performed in a state where the lock release member 46 is pressed. Although the protruding piece 411 connected to the second operating member 43 is sandwiched between the bending rib 425 and the bending rib 426, the protruding piece 411 moves with a force exceeding the frictional force. In a case in which the second operating member 43 is rotated in the direction D5 in a state where the lock release member 46 is pressed, the shape of the bendable body 30 can be slowly returned to its original linear shape.

As shown in Fig. 10, the lock release member 46 is accommodated in the accommodating member 42 in a state where a part of the lock release member 46 is exposed from an opening provided in the third surface 423. The third surface 423 has a raised portion 423A around the lock release member 46 that is raised higher than other regions. Since the lock release member 46 is provided inside the raised portion 423A, the position of the lock release member 46 can be easily grasped by the feeling of the fingers gripping the accommodating member 42. In addition, since it is necessary to move the finger onto the raised portion 423A in order to operate the lock release member 46, it is possible to reduce the probability that the lock release member 46 is erroneously operated.

As shown in Fig. 10, the lock release member 46 is provided eccentrically on the second surface 422 side in a direction in which the first surface 421 and the second surface 422 are aligned. On the other hand, the opening portion 420 is provided eccentrically on the first surface 421 side in a direction in which the first surface 421 and the second surface 422 are aligned. As described above, since the opening portion 420 and the lock release member 46 are disposed on opposite sides, it is possible to more effectively prevent the lock release member 46 from being erroneously operated.

It is preferable that the bending member 412, which is an exposed region of the rotational movement member 41, is colored in order to enable intuitive recognition of the correspondence between the operating state of the second operating member 43 and the bending state of the bendable body 30, and that the color of the exposed region is changed according to the movement position of the second operating member 43.

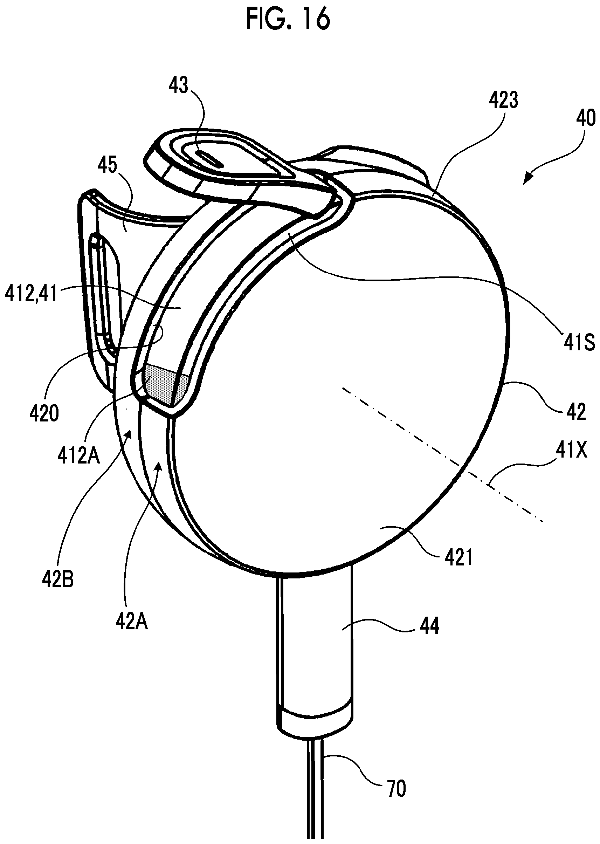

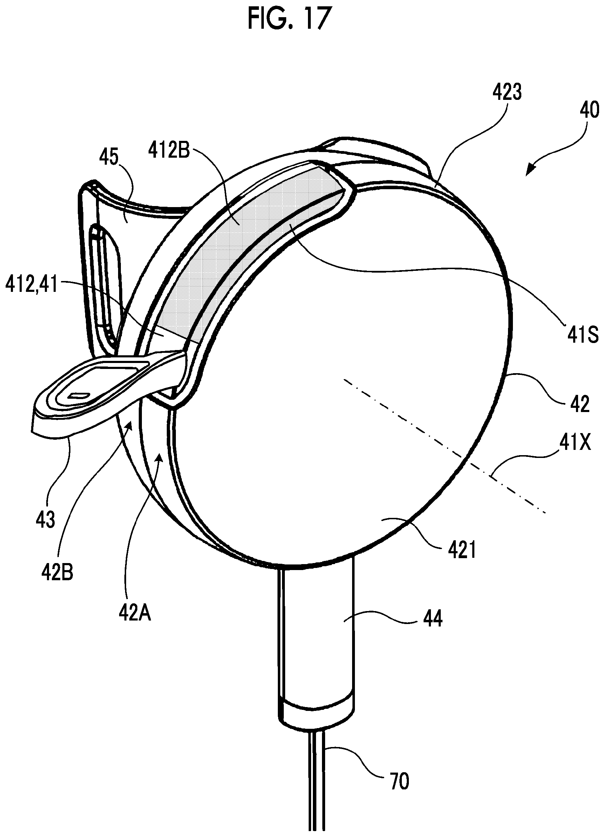

Fig. 16 is a front view of the operating device 40 in the state shown in Fig. 12. Fig. 17 is a perspective front view of the operating device 40 in the state shown in Fig. 13. In a state where the wire 71 is not pulled, a color region 412A with a first color is visible at an end part of an outer peripheral surface of the bending member 412 on a side opposite to a position where the second operating member 43 is provided.

As shown in Fig. 17, in a case in which the wire 71 is pulled, the color region 412A enters the inside of the accommodating member 42 and thus is not visible, while an outer surface of the bending member 412 hidden inside the accommodating member 42 is exposed. The outer surface is a color region 412B with a second color different from the first color.

As described above, the presence of the color region 412A and the color region 412B makes it possible to visually inform the operator whether the bendable body 30 is in the initial state or the bent state. An exposed area of the color region 412B makes it possible to visually understand how much the bendable body 30 is bent. The color region 412A and the color region 412B are attached to a front region 41S exposed on the front side of the bending member 412. As a result, visual recognition ranges of the color region 412A and the color region 412B can be widened.

In the above description, the opening portion 420 of the accommodating member 42 is formed to extend from the third surface 423 to the first surface 421, but the present invention is not limited to this. The opening portion 420 of the accommodating member 42 may be formed to extend from the third surface 423 to the second surface 422. The opening portion 420 of the accommodating member 42 may be formed to extend from the third surface 423 to the first surface 421 and the second surface 422.

In the above description, the locking part 45 is provided on the second surface 422, but the locking part 45 may be provided on the first surface 421. The reinforcing member 44 is not essential and may be omitted. Although the shape of the accommodating member 42 is a disk shape, any shape can be adopted as long as the accommodating member 42 has, on the inside of the virtual circle VC, a region that can be gripped by three or four fingers other than the thumb, which is placed on the second operating member 43, of one hand of the operator.

The configuration of the operating device 40 is not limited to a configuration of performing the bending operation of the bendable body 30 mounted on the insertion part 10, and is applicable. For example, the configuration of the operating device 40 can also be adopted to operate an elevator that is externally attached to the distal end part of the endoscope. The elevator in this case constitutes one of the endoscope auxiliary tool.

As described above, at least the following matters are described in the present specification.
(1) An operating device for an endoscope auxiliary tool, comprising:
   a first operating member having one end side connected to an endoscope auxiliary tool that is mounted on an insertion part of an endoscope;
   a rotational movement member that is rotationally movable and that is connected to the other end side of the first operating member;
   an accommodating member that accommodates the rotational movement member; and
   a second operating member that is provided to protrude from the accommodating member and that is connected to the rotational movement member,
   in which a protruding end of the second operating member is configured to be movable along a portion of a circumference of a virtual circle centered on a rotational movement axis of the rotational movement member, and
   the accommodating member has a grippable region inside the virtual circle.
(2) The operating device according to (1),
   in which the region is grippable with three or four fingers other than a thumb, which is placed on the second operating member, of one hand of an operator.
(3) The operating device according to (2),
   in which the accommodating member has a first surface on one end side in a direction of the rotational movement axis, a second surface on the other end side in the direction of the rotational movement axis, and a third surface connecting the first surface and the second surface, and
   the second operating member is provided to protrude from the third surface.
(4) The operating device according to (3),
   in which the third surface is provided with an opening portion through which the second operating member is movable.
(5) The operating device according to (4),
   in which the opening portion is provided to extend over at least one of the first surface or the second surface.
(6) The operating device according to (4) or (5),
   in which a part of the rotational movement member is exposed from the opening portion.
(7) The operating device according to (6),
   in which an exposed region of the rotational movement member is colored, and a color of the exposed region changes depending on a movement position of the second operating member.
(8) The operating device according to any one of (4) to (7),
   in which the accommodating member is provided with a lock mechanism that locks a rotational movement position of the rotational movement member in a plurality of stages,
   the opening portion is provided eccentrically on a first surface side of the third surface, and
   the third surface is provided with a lock release member that releases the lock through the lock mechanism at a position eccentrically located on a second surface side.
(9) The operating device according to any one of (3) to (8),
   in which the accommodating member is provided with a lock mechanism that locks a rotational movement position of the rotational movement member in a plurality of stages, and
   the third surface is provided with a lock release member that releases the lock through the lock mechanism and a raised portion that surrounds the lock release member.
(10) The operating device according to any one of (1) to (9),
   in which the accommodating member includes a lock mechanism that locks a rotational movement position of the rotational movement member in a plurality of stages, a lock release member that releases the lock through the lock mechanism, and a movement suppression mechanism that suppresses movement of the rotational movement member to a reference position in a case in which the lock release member is operated.
(11) The operating device according to any one of (3) to (9), further comprising:
   a locking part that is provided on any one of the first surface or the second surface and that is lockable to the endoscope.
(12) The operating device according to any one of (1) to (11),
   in which the second operating member has a curved shape as viewed in a direction of the rotational movement axis.
(13) The operating device according to any one of (1) to (12),
   in which an outer peripheral edge of the accommodating member is shaped along the virtual circle as viewed in a direction of the rotational movement axis.
(14) The operating device according to (13),
   in which the second operating member is configured to be movable in a range of 1/4 of the outer peripheral edge of the accommodating member as viewed in the direction of the rotational movement axis.
(15) The operating device according to any one of (3) to (9),
   in which a reinforcing member that reinforces a base end side of the first operating member is provided to protrude on a side opposite to the second operating member with the rotational movement axis interposed therebetween on the third surface.
(16) The operating device according to any one of (3) to (9),
   in which the first surface is configured to be larger than the second surface as viewed in the direction of the rotational movement axis, and
   the third surface is configured as a curved surface that curves from the first surface toward the second surface.
(17) The operating device according to any one of (1) to (16),
   in which an entire accommodating member is located inside the virtual circle as viewed in a direction of the rotational movement axis.

### Explanation of References

1: endoscope
   2: body part
   4: light source device
   4A: connecting portion
   5: processor device
   7: display device
   10: insertion part
   10A: soft part
   10B: bendable part
   10C, 113: distal end part
   10As: end part
   11: endoscope operating part
   11A: suction button
   12: angle knob
   13: universal cord
   13A: connector portion
   20: auxiliary device
   30: bendable body
   30S, 51: slit
   31: distal end nodal portion
   32: intermediate nodal portion
   33: base end nodal portion
   34: nodal portion
   35: coating member
   40: operating device
   41: rotational movement member
   41S: front region
   41X: rotational movement axis
   42: accommodating member
   42A: front cover member
   42B: rear cover member
   42X: circular portion
   43: second operating member
   43A: protruding end
   44: reinforcing member
   44X: rectangular portion
   45: locking part
   46: lock release member
   50: support member
   52: protrusion
   53: central protrusion
   60: locking member
   70: first operating member
   71: wire
   72: guide member
   100: endoscope apparatus
   112: treatment tool inlet port
   310, 320, 411: protruding piece
   311: engagement protrusion
   321: recess portion
   322: through-hole
   330: hole portion
   410: rotational movement axis member
   410A: ratchet groove
   412: bending member
   412A, 412B: color region
   420: opening portion
   421: first surface
   422: second surface
   423: third surface
   423A: raised portion
   424: lock member
   424A: lock piece
   425, 426: bending rib
   425A: column portion
   D1, D2, D3, D4, D5: direction
   L1, L2, L4: length
   L3: distance
   UH: hand
   F1: thumb
   F2: index finger
   F3: middle finger
   F4: ring finger
   F5: little finger

## Claims

1. An operating device for an endoscope auxiliary tool, comprising:
a first operating member having one end side connected to an endoscope auxiliary tool that is mounted on an insertion part of an endoscope;
a rotational movement member that is rotationally movable and that is connected to the other end side of the first operating member;
an accommodating member configured to accommodate the rotational movement member; and
a second operating member that is provided to protrude from the accommodating member and that is connected to the rotational movement member,
wherein a protruding end of the second operating member is configured to be movable along a portion of a circumference of a virtual circle centered on a rotational movement axis of the rotational movement member, and
the accommodating member has a grippable region inside the virtual circle.

2. The operating device according to claim 1,
wherein the region is grippable with three or four fingers other than a thumb, which is placed on the second operating member, of one hand of an operator.

3. The operating device according to claim 2,
wherein the accommodating member has a first surface on one end side in a direction of the rotational movement axis, a second surface on the other end side in the direction of the rotational movement axis, and a third surface connecting the first surface and the second surface, and
the second operating member is provided to protrude from the third surface.

4. The operating device according to claim 3,
wherein the third surface has an opening portion through which the second operating member is movable.

5. The operating device according to claim 4,
wherein the opening portion is provided to extend over at least one of the first surface or the second surface.

6. The operating device according to claim 4 or 5,
wherein a part of the rotational movement member is exposed from the opening portion.

7. The operating device according to claim 6,
wherein an exposed region of the rotational movement member is colored, and
a color of the exposed region changes depending on a movement position of the second operating member.

8. The operating device according to any one of claims 4 to 7,
wherein the accommodating member has a lock mechanism configured to lock a rotational movement position of the rotational movement member in a plurality of stages,
the opening portion is provided eccentrically on a first surface side of the third surface, and
the third surface has a lock release member configured to release the lock through the lock mechanism at a position eccentrically located on a second surface side.

9. The operating device according to any one of claims 3 to 8,
wherein the accommodating member has a lock mechanism configured to lock a rotational movement position of the rotational movement member in a plurality of stages, and
the third surface has a lock release member configured to release the lock through the lock mechanism and a raised portion that surrounds the lock release member.

10. The operating device according to any one of claims 1 to 9,
wherein the accommodating member includes a lock mechanism configured to lock a rotational movement position of the rotational movement member in a plurality of stages, a lock release member configured to release the lock through the lock mechanism, and a movement suppression mechanism configured to suppress movement of the rotational movement member to a reference position in a case in which the lock release member is operated.

11. The operating device according to any one of claims 3 to 9, further comprising:
a locking part that is provided on any one of the first surface or the second surface and that is lockable to the endoscope.

12. The operating device according to any one of claims 1 to 11,
wherein the second operating member has a curved shape as viewed in a direction of the rotational movement axis.

13. The operating device according to any one of claims 1 to 12,
wherein an outer peripheral edge of the accommodating member is shaped along the virtual circle as viewed in a direction of the rotational movement axis.

14. The operating device according to claim 13,
wherein the second operating member is configured to be movable in a range of 1/4 of the outer peripheral edge of the accommodating member as viewed in the direction of the rotational movement axis.

15. The operating device according to any one of claims 3 to 9,
wherein a reinforcing member that reinforces a base end side of the first operating member is provided to protrude on a side opposite to the second operating member with the rotational movement axis interposed therebetween on the third surface.

16. The operating device according to any one of claims 3 to 9,
wherein the first surface is configured to be larger than the second surface as viewed in the direction of the rotational movement axis, and
the third surface is configured as a curved surface that curves from the first surface toward the second surface.

17. The operating device according to any one of claims 1 to 16,
wherein an entire accommodating member is located inside the virtual circle as viewed in a direction of the rotational movement axis.
